# EUROPEAN PATENT SPECIFICATION

(11) **EP 2 785 203 B1**
(45) Date of publication and mention of the grant of the patent: **07.03.2018**
(21) Application number: 12790898.6
(22) Date of filing: 22.11.2012
(51) Int. Cl.: A61K 31/33, A61P 37/04, A61P 39/06, A23K 20/105, A23K 50/40, A23L 33/105, A61K 31/192, A61K 31/353, A61K 31/7048, A61K 47/26, A61K 8/365, A61K 8/60, A61Q 19/00, A61Q 19/08

(54) **INCREASING THE BIOAVAILABILITY OF HYDROXYCINNAMIC ACIDS**
ERHÖHEN DER VERFÜGBARKEIT VON HYDROXYZIMTSÄUREN
AUGMENTATION DE LA BIODISPONIBILITÉ D'ACIDES HYDROXYCINNAMIQUES

(30) Priority: 28.11.2011 EP 11190863
(43) Date of publication of application: 08.10.2014
(73) Proprietor: Nestec S.A., 1800 Vevey (CH)
(72) Inventor: RENOUF, Mathieu, CH-1814 La Tour-de-Peilz (CH); WILLIAMSON, Gary, Harrogate Yorkshire HG2 7AX (GB); DIONISI, Fabiola, CH-1066 Epalinges (CH); POQUET, Laure, CH-1077 Servion (CH)
(74) Representative: Naudé, Dawn
(86) International application number: PCT/EP2012/073383
(87) International publication number: WO 2013/079394

(56) References cited:
- WO-A1-2011/140655
- CABRERA C ET AL: "Beneficial effects of green tea - A review", JOURNAL OF THE AMERICAN COLLEGE OF NUTRITION, AMERICAN COLLEGE OF NUTRION, WILMINGTON, NC, US, vol. 25, no. 2, 30 April 2006 (2006-04-30) , pages 79-99, XP003021660, ISSN: 0731-5724
- Laurent Y Rios ET AL: "Chocolate intake increases urinary excretion of polyphenol-derived phenolic acids in healthy human subjects", American Journal of Clinical Nutrition, 30 April 2003 (2003-04-30), pages 912-918, XP055024496, Retrieved from the Internet: URL:http://www.ajcn.org/content/77/4/912.f ull.pdf+html [retrieved on 2012-04-16]
- ALENA SVOBODOV ET AL: "Natural phenolics in the prevention of UV-induced skin damage. A review.", BIOMEDICAL PAPERS OF THE FACULTY OF MEDICINE OF PALACKÝ UNIVERSITY, OLOMOUC CZECH REPUBLIC, vol. 147, no. 2, 31 December 2003 (2003-12-31), pages 137-145, XP055024497, ISSN: 1213-8118
- SILBERBERG M ET AL: "The bioavailability of polyphenols is highly governed by the capacity of the intestine and of the liver to secrete conjugated metabolites", EUROPEAN JOURNAL OF NUTRITION, STEINKOPFF-VERLAG, DA, vol. 45, no. 2, 30 June 2005 (2005-06-30), pages 88-96, XP019383110, ISSN: 1436-6215, DOI: 10.1007/S00394-005-0568-5
- Jane Higdon, Victoria J. Drake, Roderick H. Dashwood: "Flavonoids", Oregon State University Linus Pauling Institute, Micronutrient Research for Optimum Health, 30 June 2008 (2008-06-30), pages 1-9, XP002696591, http://lpi.oregonstate.edu Retrieved from the Internet: URL:http://lpi.oregonstate.edu/infocenter/ phytochemicals/flavonoids/ [retrieved on 2013-05-07]
- ZHAOHUI ZHAO ET AL: "Bioavailability of hydroxycinnamates: a brief review of in vivo and in vitro studies", PHYTOCHEMISTRY REVIEWS, KLUWER ACADEMIC PUBLISHERS, DO, vol. 9, no. 1, 30 September 2009 (2009-09-30), pages 133-145, XP019794057, ISSN: 1572-980X
- SUSANNE SCHOLZ AND GARY WILLIAMSON: "Interactions affecting the bioavailability of dietary polyphenols in vivo", INT J VITAM NUTR RES, vol. 77, no. 3, May 2007 (2007-05), pages 224-235, XP009158407,
- MANACH C ET AL: "Polyphenols: food sources and bioavailability", THE AMERICAN JOURNAL OF CLINICAL NUTRITION, AMERICAN SOCIETY FOR NUTRITION, US, vol. 79, no. 5, 1 January 2004 (2004-01-01), pages 727-747, XP002521676, ISSN: 0002-9165
- Heather Boon ET AL: "Quercetin", Natural Standard Monograph, 1 January 2008 (2008-01-01), pages 1-20, XP055194357, Retrieved from the Internet: URL:http://www.natrodale.co.za/uploads/Nat rodale_Arthrosmart_Quercetin.pdf [retrieved on 2015-06-09]
- PAGANGA G ET AL: "THE POLYPHENOLIC CONTENT OF FRUIT AND VEGETABLES AND THEIR ANTIOXIDANT ACTIVITIES. WHAT DOES A SERVING CONSTITUTE?", FREE RADICAL RESEARCH, YVERDON, CH, vol. 30, no. 2, 1 January 1999 (1999-01-01), pages 153-162, XP000918290, ISSN: 1071-5762
- Gary Williamson ET AL: "Flavanols from green tea and phenolic acids from coffee: Critical quantitative evaluation of the pharmacokinetic data in humans after consumption of single doses of beverages", MOLECULAR NUTRITION & FOOD RESEARCH, vol. 55, no. 6, 2 May 2011 (2011-05-02), pages 864-873, XP055386941, DE ISSN: 1613-4125, DOI: 10.1002/mnfr.201000631
- Rita C. Alves ET AL: "Isoflavones in Coffee: Influence of Species, Roast Degree, and Brewing Method", Journal of Agricultural and Food Chemistry, vol. 58, no. 5, 10 March 2010 (2010-03-10) , pages 3002-3007, XP055387263, ISSN: 0021-8561, DOI: 10.1021/jf9039205

## Description

The present invention generally relates to the field of nutrition, health and wellness. For example, the present invention relates to hydroxycinnamic acids and their health benefits. The present invention discloses compositions that allow increasing the bioavailability and/or bioefficacy of hydroxycinnamic acids. According to the invention, this can be achieved by co-administering at least one glycoside conjugate of a flavonoid with hydroxycinnamic acids.

Hydroxycinnamic acids are highly abundant phenolic compounds in our diet, and they are ubiquitously found in fruits, vegetables and coffee. Estimates of daily intake of hydroxycinnamic acids could be very high (500-1000 mg/d), especially among coffee drinkers. In vitro and in vivo studies have suggested that the consumption of hydroxycinnamic acids is associated with beneficial effects linked to their antioxidant capacity [Natella F, et al., J Agric Food Chem 2002;50:6211-6; Natella F, et al., Am J Clin Nutr 2007;86:604-9; Poquet L, et al., Arch Biochem Biophys 2008;476:196-204.].

However, in humans, the first-pass metabolism of hydroxycinnamic acids plays a major role in limiting their bioavailability. Phase II enzymes in particular, are directly involved in the inactivation of dietary hydroxycinnamic acids by forming conjugates of sulfate, glucuronide or amino acid [Poquet L, et al., Biochem Pharmacol 2008;75:1218-29]. Several human and in vitro mechanistic studies showed that absorbed hydroxycinnamates are extensively metabolized in the intestine and liver, and identified sulfotransferases (SULTs) as the major metabolic enzymes involved. As a consequence, sulfate conjugates of hydroxycinnamic acids are the major forms detected in human plasma and urine, while the free acids are found in low levels. In humans, cytosolic SULTs consist of 11 members that catalyze the sulfation of low molecular weight endogenous compounds and xenobiotics [Blanchard RL et al., Pharmacogenetics 2004;14:199-211]. Hydroxycinnamic acid sulfates are the major products formed in the human liver and intestinal homogenates, indicating that both organs contributed to hydroxycinnamic acid sulfation in humans. Sulfation is targeted at the phenolic hydroxyl groups, which are a major determinant of the strong antioxidant capacity of hydroxycinnamic acids [Giacomelli C, et al., Redox Rep 2004;9:263-9]. Hence, sulfation of hydroxycinnamic acids has a significant impact on their bioavailability and reducing their bioefficacy.

As, however, free hydroxycinnamic acids exhibit the beneficial antioxidant capacity, it would be desirable to have available a composition that allows to increase the bioavailability of hydroxycinnamic acids.

The present inventors have addressed this need.

It was consequently the objective of the present invention to improve the state of the art and to provide a natural composition that allows increasing the bioavailability of hydroxycinnamic acids and that consequently exhibits an improved antioxidant capacity.

The present inventors were surprised to see that they could achieve this objective by the subject matter of the independent claims. The dependant claims further develop the idea of the present invention.

The inventors have demonstrated the inhibitory effects of flavonoids and their conjugates on the sulfation of five major dietary hydroxycinnamic acids (caffeic, dihydrocaffeic, dihydroferulic, ferulic and isoferulic acids).

Inhibitory effects of eleven dietary flavonoids aglycones on sulfation of hydroxycinnamic acids were shown in human intestine and liver homogenates. The effect of quercetin-3-glucuronide, quercetin-7-glucuronide and quercetin-3'-sulfate, the major quercetin conjugates in human plasma, on sulfation was shown in human liver homogenates.

The inhibitory effect of luteolin, quercetin and quercetin conjugates on hydroxycinnamic acids sulfation was also demonstrated in the human hepatoma cell line, HepG2, as a model for human liver.

Based on these findings, the inventors believe that the inhibition of SULTs is a possible strategy to improve the bioavailability of unconjugated hydroxycinnamic acids, which in turn, leads to an enhanced bioefficacy. In particular, human gut SULTs may be inhibited by dietary doses of flavonoids, since the local concentrations of flavonoids in gut is much higher than that in plasma [Manach C, et al., Am J Clin Nutr 2005;81:230S-42S].

Human liver is another important site of hydroxycinnamic acid sulfation. However, in vivo, the flavonoids are extensively metabolized into conjugates.

The inventors have now found that flavonoid conjugates are also effective inhibitors of sulfation of hydroxycinnamic acids after intestinal absorption.

With flavonoids having the potential to modulate the bioavailability of hydroxycinnamic acids via the inhibition of SULT1A in the human intestine and liver, the co-consumption of hydroxycinnamic acids together with flavonoids allows an increase of the concentration of unconjugated hydroxycinnamic acids in the circulation, and as a consequence an increase of the bioefficacy following the ingestion of hydroxycinnamic acids.

Consequently, the present invention relates to a composition for use in the treatment, alleviation or prevention of disorders linked to oxidative stress, inflammatory processes and/or a reduced immune response, comprising at least one dietary hydroxycinnamic acid and comprising genistein, daidzein, apigenin, phloretin, luteolin, quercetin in combination and wherein at least one glycoside conjugate of a flavonoid is added to the hydroxycinnamic acid containing composition, and wherein the dietary hydroxycinnamic acid is a combination of caffeic acid, dihydrocaffeic acid, dihydroferulic acid, ferulic acid, and isoferulic acid, and wherein the glycoside conjugate of a flavonoid is selected from the group consisting of glycoside conjugates of quercetin.
For example, the content of the at least one glycoside conjugate of a flavonoid and/or the at least one hydroxycinnamic acids may be enriched by a factor of 1.2, 1.5, 1.7, 2, 5, or 10,

The at least one glycoside conjugate of a flavonoid will then increase the bioavailability and bioefficacy of the hydroxycinnamic acids that are consumed with the daily nutrition.

The inventors have found that the at least one glycoside conjugate of a flavonoid inhibits at least partially the sulfation of dietary hydroxycinnamic acids.

The inventors found genistein, daidzein, apigenin, phloretin, luteolin and quercetin were particularly effective under the conditions tested.

Importantly, glycoside conjugates of quercetin are the glycoside conjugates of a flavonoid used for the purpose of the present invention.

As glycosides, rhamnose, glucose, galactose, xylose, arabinose, fucose or combinations of theses glycosides, for example rutinoside may be used.
Examples of glycoside conjugates of flavonoids may be rutin (quercetin-3-0-rutinoside).
In therapeutic applications, compositions are administered in an amount sufficient to at least partially cure or arrest the symptoms of the disease and its complications. An amount adequate to accomplish this is defined as "a therapeutically effective dose". Amounts effective for this purpose will depend on a number of factors known to those of skill in the art such as the severity of the disease and the weight and general state of the patient.
In prophylactic applications, compositions according to the invention are administered to a patient susceptible to or otherwise at risk of a particular disease in an amount that is sufficient to at least partially reduce the risk of developing a disease. Such an amount is defined to be "a prophylactic effective dose". Again, the precise amounts depend on a number of patient specific factors such as the patient's state of health and weight.

For example, the composition of the present invention may comprise at least 1mg hydroxycinnamic acid, at least 10mg hydroxycinnamic acid, or at least SOmg hydroxycinnamic acid per serving.
For example, the composition of the present invention may comprise at least 10 mg, at least 100 mg, or at least 1000mg flavonoids and/or their conjugates per serving.

The at least one glycoside conjugate of a flavonoid may be provided in any form, e.g., as chemically synthesized compounds or as compounds purified from natural sources.

It is however preferred that the at least one glycoside conjugate of a flavonoid added to the composition in the form of a natural food product or an extract thereof. This will underline the naturality of the composition.

Natural sources of the at least one glycoside conjugate of a flavonoid may be selected from the group consisting of black or green tea, capers, lovage, apples, onion, in particular red onion, red grapes, citrus fruit, tomato, broccoli, raspberry, bog whortleberry, lingonberry, cranberry, chokeberry, sweet rowan, rowanberry, sea buckthorn berry, crowberry, prickly pear cactus fruit, or combinations thereof.
Similarly, also the hydroxycinnamic acid may be provided in any form, e.g., as chemically synthesized compounds or as compounds purified from natural sources. Also here it is preferred if the hydroxycinnamic acid is provided as natural food product or extract thereof.
For example, hydroxycinnamic acids may be obtained from or provided as coffee, cocoa, vegetables or fruits.
The hydroxycinnamic acid is a dietary hydroxycinnamic acid consisting of a combination of caffeic acid, dihydrocaffeic acid, dihydroferulic acid, ferulic acid, isoferulic acid.

The composition of the present invention is for use in the treatment, prevention or alleviation of disorders linked to oxidative stress, inflammatory processes and/or a reduced immune response.
These disorders may be selected from the group consisting of hyperglycemia, type 2 diabetes mellitus, cardiovascular disorders, acute immune and/or inflammatory responses, skin cells damage caused by ultraviolet (UV) radiation, accelerated cell aging, and combinations thereof.

The composition may be any composition suitable for human or animal use. For example, the composition may be selected from the group consisting of food products, drinks, pet food products, nutraceuticals, food additives or cosmetic products.

As such, the composition may be to be administered to humans or animals, for example pet animals such as cats or dogs. The composition may be consumed at any time of the day. It may be preferred, however, to administer the compositions of the present invention in the morning to prepare the body for the challenges of the day.

To ensure a good protection of the body throughout the days, it may also be preferred to consume the composition of the present invention also during the day, for example with the meals. As such the compositions of the present invention may be to be administered in the morning, at lunchtime and in the evening.

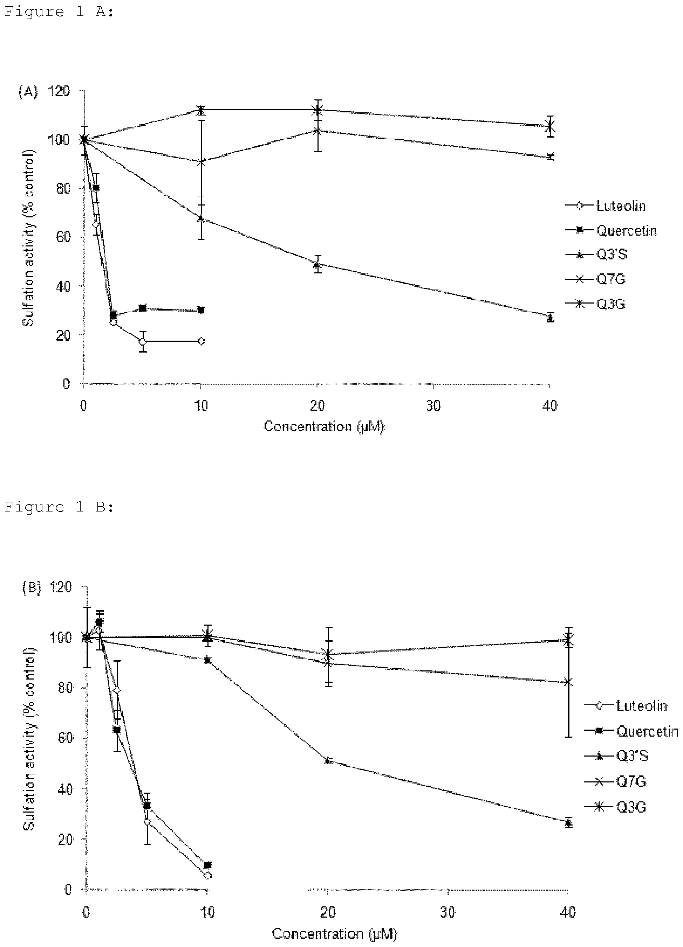

Further advantages and features of the present invention are apparent from the following Comparative Examples and Figures. Figure 1A and B shows the inhibitory effect of luteolin, quercetin, and quercetin conjugates on sulfation of caffeic acid (A) and ferulic acid (B) in HepG2 cells. Caffeic and ferulic acids (10 µM) were incubated in the presence of luteolin, quercetin or quercetin conjugates for 4 h.

### Comparative Examples:

### 1.1. Inhibition of hydroxycinnamic acids sulfation by flavonoids in human liver and intestinal S9

The incubation mixture, in a final volume of 50 µL, consisted of 100 mM potassium phosphate buffer (pH 7.4), with 100 µM vitamin C, 100 µM PAPS and 1 mM DTT. Human liver S9 and intestinal S9 homogenates were used at 1 mg/mL and 0.4 mg/mL, respectively. The flavonoids were added from a 5 mM stock solution dissolved in DMSO, with final DMSO concentration equalized to 0.2 %. Quercetin-3-glucuronide, quercetin-7-glucuronide and quercetin-3'-sulfate were dissolved in water. After a 15 min pre-incubation period, the reaction was initiated by adding 10 µM cinnamic acids and 25 µM dihydrocinnamic acids from a 50 mM stock solution in DMSO. To inhibit hydrolysis of quercetin glucuronides, in some analyses, 5 mM saccharolactone was added. After 30 min incubation in a 37 °C water bath, the reaction was stopped by addition of 10 µL ice-cold acetonitrile containing 500 mM HCl. Controls were treated under identical condition and consisted of samples with 0.2 % DMSO (final concentration) added to the buffer. Samples were stored at -70 °C until analysis.

### 1.2. HepG2 Cell culture

HepG2 cells (ATCC) were routinely cultured in 75 cm" cell culture flasks at 37 QC under a humidified 5 % CO2 / O2 atmosphere. The culture media consisted of Eagle's Minimum Essential Medium (EMEM) media supplemented with 10% fetal bovine serum (Sigma-Aldrich) and 100U/ml penicillin-streptomycin. All experiments were performed with HepG2 cells between passages 80 to 95. For metabolic studies, HepG2 cells were seeded into 12-well plates at a cell density of 2 x 10⁵ per well. The cell monolayers were allowed to grow over 96 h before they were used for experiments. Hydroxycinnamic acid metabolism experiments were carried out in serum-free media with 100 µM vitamin C and 1.8 mM CaC12, adjusted to pH 6.5. Cinnamic acids (10 µM) and dihydrocinnamic acids (25 µM) were added from a 50 mM stock solution in DMSO. Quercetin and luteolin 15 mM) were also dissolved in DMSO and added to the media to give a final DMSO concentration of 0.25 %. 0.4 mL of hydroxycinnamic acids, with or without the inhibitors, were added to the HepG2 cells and incubated for 4 h at 37 QC. The incubation media were then collected, acidified with 1 mM vitamin C and dried under vacuum. The residue was extracted by sonication for 5 min and vortex for 1 min, first with 500 µL acetonitrile, followed by 500 µL methanol. The extracts were combined and centrifuged at 17,000 g for 10 min. The supernatant was evaporated under vacuum. Prior to HPLC analysis, the dried residue was re-dissolved in 100 µL of initial mobile phase.

### 1.3. HPLC methodology for hydroxycinnamic acids

HPLC analyses were carried out using the Agilent 1200 series liquid chromatography system. For the analysis of caffeic acid, ferulic acid, isoferulic acid, dihydroferulic acid and their conjugates, chromatography was performed with a Zorbax XDB-C18 column (4.6 x 150 mm, 5 µm). The mobile phase consisted of 20 mM ammonium formate, pH 2.8 (A) and methanol (B). For the analysis of caffeic acid and conjugates, samples were eluted at 1 mL/min with 5 % to 25 % B in 20 min, followed by 80 % B in 2 min and back to 5 % B for 3 min. For the analysis of ferulic and isoferulic acid and their conjugates, the gradient was from 10 % to 20 % B in 10 min, to 60% B in 15 min, then set at 80 % B for 2 min and back to 10 % B for 3 min, at 1 mL/min. Dihydroferulic acid and conjugates analysis was carried out at 1 mL/min, from 10 % to 20 % B in 15 min, to 60 % B in 10 min, up to 80 % B for 2 min and finally to 10 % B for 3 min. For dihydrocaffeic acid and conjugates, the analyses were performed with a Zorbax XDB-C18 column (4.6 x 50 mm, 1.8 µm) with 20 mM ammonium formate, pH 4.5 (A) and methanol (B) as the mobile phase. The gradient started at 3 % (B) kept for 15 min, followed by an increase to 40 % B in 5 min, and then returned to 3 % B for 5 min. Samples were centrifuged and 25 µL of the supernatant were injected into the column. UV-detection carried out at 280 nm and 310 nm using photodiode array detector. Caffeic, ferulic and isoferulic acid were quantified at 310 nm, dihydroferulic acid and dihydrocaffeic acid at 280 nm.

### 2. Results

The inhibitory effect (IC₅₀) of flavonoids and their conjugates is shown in Table 1, 2 and 3.

**Table 1. Inhibition of caffeic acid and ferulic acid sulfation by flavonoids in human intestinal S9**

| Flavonoids | IC₅₀ (µM) for the inhibition of sulfation of | |
|---|---|---|
| | Caffeic acid | Ferulic acid |
| Apigenin | 0.96 | 2.3 |
| Luteolin | 1.3 | 3.0 |
| Kaempferol | 2.5 | 3.8 |
| Isorhamnetin | 4.4 | 4.7 |
| Quercetin | 4.2 | 5.2 |
| Hesperetin | 3.5 | 5.1 |
| Genistein | 0.77 | 1.5 |
| Daidzein | 0.72 | 2.3 |
| (+)-Catechin | 5.8 | 7.0 |
| (-)-Epicatechin | 7.6 | 11 |
| Phloretin | 0.84 | 2.3 |

**Table 2. Inhibition of caffeic acid and ferulic acid sulfation by flavonoids in human liver S9**

| Flavonoids | IC₅₀ (µM) for the inhibition of sulfation of | |
|---|---|---|
| | Caffeic acid | Ferulic acid |
| Apigenin | 0.46 | 0.88 |
| Luteolin | 0.08 | 0.53 |
| Kaempferol | 0.92 | 3.9 |
| Isorhamnetin | 2.0 | 3.7 |
| Quercetin | 0.41 | 0.64 |
| Hesperetin | 4.3 | 5.3 |
| Genistein | 0.59 | 1.0 |
| Daidzein | 0.65 | 0.62 |
| (+)-Catechin | 4.2 | 4.4 |
| (-)-Epicatechin | 7.4 | 7.3 |
| Phloretin | 0.99 | 1.0 |

**Table 3. Inhibition of caffeic acid and ferulic acid sulfation by quercetin conjugates in liver S9**

| Conjugate | IC₅₀ (µM) for the inhibition of sulfation of | |
|---|---|---|
| | Caffeic acid | Ferulic acid |
| Quercetin-3-glucuronide | 11 | 12 |
| Quercetin-7-glucuronide | 9.6 | 8.0 |
| Quercetin-3'-sulfate | 6.6 | 6.7 |

Flavonoids were found to be potent inhibitors of sulfation of hydroxycinnamic acid. Isoflavones were the strongest inhibitors in intestinal S9, while quercetin and luteolin were the most effective inhibitors in liver S9. Quercetin conjugates, as the forms found in blood, were also effective, with IC50 values in the low micro-molar range.

The effect of flavonoids and conjugates on the sulfation of hydroxycinnamic acids in HepG2 cells was summarized in Figure 1A and 1B.

## Claims

1. Composition for use in the treatment, alleviation or prevention of disorders linked to oxidative stress, inflammatory processes and/or a reduced immune response, comprising at least one dietary hydroxycinnamic acid and comprising genistein, daidzein, apigenin, phloretin, luteolin, quercetin in combination and wherein at least one glycoside conjugate of a flavonoid is added to the hydroxycinnamic acid containing composition, and wherein the dietary hydroxycinnamic acid is a combination of caffeic acid, dihydrocaffeic acid, dihydroferulic acid, ferulic acid, and isoferulic acid, and wherein the glycoside conjugate of a flavonoid is selected from the group consisting of glycoside conjugates of quercetin.

2. Composition for use in accordance with claim 1, wherein the at least one glycoside conjugate of a flavonoid are added to the composition in the form of a natural food product or an extract thereof.

3. Composition for use in accordance with claim 2, wherein the natural food is selected from the group consisting of black or green tea, capers, lovage, apples, onion, in particular red onion, red grapes, citrus fruit, tomato, broccoli, raspberry, bog whortleberry, lingonberry, cranberry, chokeberry, sweet rowan, rowanberry, sea buckthorn berry, crowberry, prickly pear cactus fruit, or combinations thereof.

4. Composition for use in accordance with one of the preceding claims, wherein disorders linked to oxidative stress, inflammatory processes and/or a reduced immune response are selected from the group consisting of hyperglycemia, type 2 diabetes mellitus, cardiovascular disorders, acute immune and/or inflammatory responses, skin cells damage caused by ultraviolet (UV) radiation, accelerated cell aging, and combinations thereof.

5. Composition for use in accordance with one of the proceeding claims, wherein the composition is selected from the group consisting of food products, drinks, petfood products, nutraceuticals, food additives or cosmetic products.

6. Composition for use in accordance with one of the proceeding claims to be administered in the morning.

7. Composition for use in the treatment or prevention of oxidative damage for cosmetic purposes comprising at least one dietary hydroxycinnamic acid and comprising genistein, daidzein, apigenin, phloretin, luteolin, quercetin in combination and at least one glycoside conjugate of a flavonoid, wherein the composition is to be administered orally and wherein, the composition is enriched in at least one glycoside conjugate of a flavonoid and/or at least one hydroxycinnamic acid, compared to the natural content of glycoside conjugates of flavonoids and/or hydroxycinnamic acids of said composition, wherein the dietary hydroxycinnamic acid is a combination of caffeic acid, dihydrocaffeic acid, dihydroferulic acid, ferulic acid, and isoferulic acid, and wherein the glycoside conjugate of a flavonoid is selected from the group consisting of glycoside conjugates of quercetin.

## Patentansprüche

1. Zusammensetzung zur Verwendung bei der Behandlung, Linderung oder Vorbeugung von Störungen, die mit oxidativem Stress, Entzündungsprozessen und/oder einer verminderten Immunantwort verbunden sind, umfassend mindestens eine Ernährungs-Hydroxyzimtsäure und umfassend Genistein, Daidzein, Apigenin, Phloretin, Luteolin, Quercetin in Kombination, und wobei mindestens ein Glycosidkonjugat eines Flavonoids zu der Hydroxyzimtsäure enthaltenden Zusammensetzung gegeben wird, und wobei die Ernährungs-Hydroxyzimtsäure eine Kombination aus Kaffeesäure, Dihydrokoffeinsäure, Dihydroferulasäure, Ferulasäure und Isoferulasäure ist, und wobei das Glycosidkonjugat eines Flavonoids ausgewählt ist aus der Gruppe, bestehend aus Glycosidkonjugaten von Quercetin.

2. Zusammensetzung zur Verwendung nach Anspruch 1, wobei das mindestens eine Glycosidkonjugat eines Flavonoids der Zusammensetzung in Form eines natürlichen Nahrungsmittelprodukts oder eines Extrakts davon zugesetzt wird.

3. Zusammensetzung zur Verwendung nach Anspruch 2, wobei das natürliche Lebensmittel ausgewählt ist aus der Gruppe, bestehend aus schwarzem oder grünem Tee, Kapern, Liebstöckel, Äpfeln, Zwiebeln, insbesondere roten Zwiebeln, roten Trauben, Zitrusfrüchten, Tomaten, Brokkoli, Himbeere, Rauschbeere, Preiselbeere, Moosbeere, Apfelbeere, süße Eberesche, Vogelbeere, Sanddornbeere, Schwarze Krähenbeere, Kaktusfrucht oder Kombinationen davon.

4. Zusammensetzung zur Verwendung nach einem der vorhergehenden Ansprüche, wobei Störungen, die mit oxidativem Stress, Entzündungsprozessen und/oder einer verminderten Immunantwort verbunden sind, ausgewählt sind aus der Gruppe, bestehend aus Hyperglykämie, Diabetes mellitus Typ 2, Herz-Kreislaufstörungen, akuten Immunsystem- und/oder Entzündungsreaktionen, Hautzellenschädigungen durch ultraviolette (UV) Strahlung, beschleunigte Zellalterung und Kombinationen davon.

5. Zusammensetzung zur Verwendung gemäß einem der vorhergehenden Ansprüche, wobei die Zusammensetzung ausgewählt ist aus der Gruppe, bestehend aus Nahrungsmittelprodukten, Getränken, Heimtierfutterprodukten, Nutrazeutika, Lebensmittelzusatzstoffen oder kosmetischen Produkten.

6. Zusammensetzung zur Verwendung gemäß einem der vorstehenden Ansprüche, die am Morgen zu verabreichen ist.

7. Zusammensetzung zur Verwendung bei der Behandlung oder Vorbeugung von oxidativer Schädigung für kosmetische Zwecke, umfassend mindestens eine Ernährungs-Hydroxyzimtsäure und umfassend Genistein, Daidzein, Apigenin, Phloretin, Luteolin, Quercetin in Kombination und mindestens ein Glycosidkonjugat eines Flavonoids, wobei die Zusammensetzung oral zu verabreichen ist, und wobei die Zusammensetzung verglichen mit dem natürlichen Gehalt an Glycosidkonjugaten in Flavonoiden und/oder Hydroxyzimtsäuren der Zusammensetzung in mindestens einem Glycosidkonjugat eines Flavonoids und/oder mindestens einer Hydroxyzimtsäure angereichert ist, wobei die Ernährungs-Hydroxyzimtsäure eine Kombination aus Kaffeesäure, Dihydrokoffeinsäure, Dihydroferulasäure, Ferulasäure und Isoferulasäure ist, und wobei das Glycosidkonjugat eines Flavonoids ausgewählt ist aus der Gruppe, bestehend aus Glycosidkonjugaten von Quercetin.

## Revendications

1. Composition utilisable dans le traitement, l'atténuation ou la prévention de troubles liés au stress oxydatif, à des processus inflammatoires et/ou à la réduction de la réponse immunitaire, comprenant au moins un acide hydroxycinnamique alimentaire et comprenant de la génistéine, daidzéine, apigénine, phlorétine, lutéoline, quercétine en combinaison et dans laquelle au moins un conjugué de glycoside d'un flavonoïde est ajouté à la composition contenant l'acide hydroxycinnamique et l'acide hydroxycinnamique alimentaire est une combinaison d'acide caféique, acide dihydrocaféique, acide dihydroférulique, acide férulique et acide isoférulique et le conjugué de glycoside d'un flavonoïde est choisi dans le groupe constitué de conjugués de glycoside de quercétine.

2. Composition utilisable selon la revendication 1, dans laquelle le ou les conjugués de glycoside d'un flavonoïde sont ajoutés à la composition sous la forme d'un produit alimentaire naturel ou d'un extrait de celui-ci.

3. Composition utilisable selon la revendication 2, dans laquelle l'aliment naturel est choisi dans le groupe constitué de thé noir ou vert, câpres, livèche, pommes, oignon, en particulier l'oignon rouge, raisin rouge, agrumes, tomate, brocolis, framboise, airelle des marécages, airelle rouge, canneberge, aronia, sorbier doux, sorbes, baies de l'argousier, camarine, fruit du cactus (figue de barbarie) ou leurs combinaisons.

4. Composition utilisable selon une des revendications précédentes, dans laquelle les troubles liés au stress oxydatif, à des processus inflammatoires et/ou à la réduction de la réponse immunitaire sont choisis dans le groupe constitué de l'hyperglycémie, le diabète de type 2, des troubles cardiovasculaires, des réactions inflammatoires et/ou immunitaires aiguës, des lésions des cellules cutanées provoquées par un rayonnement ultraviolet (UV), un vieillissement accéléré des cellules et leurs combinaisons

5. Composition utilisable selon l'une des revendications précédentes, où la composition est choisie dans le groupe constitué de produits alimentaires, boissons, produits alimentaires pour animaux de compagnie, produits nutraceutiques, additifs alimentaires ou produits cosmétiques.

6. Composition utilisable selon l'une des revendications précédentes, destinée à être administrée le matin.

7. Composition utilisable dans le traitement ou la prévention de dommage oxydatif à des fins cosmétiques, comprenant au moins un acide hydroxycinnamique alimentaire et comprenant de la génistéine, daidzéine, apigénine, phlorétine, lutéoline, quercétine en combinaison et au moins un conjugué de glycoside d'un flavonoïde, où la composition est destinée à être administrée par voie orale et la composition est enrichie en au moins un conjugué de glycoside d'un flavonoïde et/ou au moins un acide hydroxycinnamique, par comparaison avec la teneur naturelle de conjugués de glycoside de flavonoïdes et/ou d'acides hydroxycinnamiques de ladite composition, dans laquelle l'acide hydroxycinnamique alimentaire est une combinaison d'acide caféique, acide dihydrocaféique, acide dihydroférulique, acide férulique et acide isoférulique et dans laquelle le conjugué de glycoside d'un flavonoïde est choisi dans le groupe constitué de conjugués de glycoside de quercétine.
